# EUROPEAN PATENT APPLICATION

(11) **EP 3 051 451 A1**
(43) Date of publication of application: **03.08.2016**
(21) Application number: 16152406.1
(22) Date of filing: 22.01.2016
(51) Int. Cl.: G06F 21/32, G06F 21/62, G06F 19/00, A61B 5/00, H04L 29/06, H04W 12/02

(54) **A MEDICAL DATA RECORDING DEVICE AND A SYSTEM FOR MEDICAL DATA STORAGE AND DISTRIBUTION**

(30) Priority: 28.01.2015 PL 41108515
(71) Applicant: Podlesny, Piotr, 32-088 Grebynice (PL); Mossakowski, Marek, 41-218 Sosnowiec (PL); Ciolczyk, Robert, 32-543 Myslachowice (PL)
(72) Inventor: Podlesny, Piotr, 32-088 Grebynice (PL); Mossakowski, Marek, 41-218 Sosnowiec (PL); Ciolczyk, Robert, 32-543 Myslachowice (PL)
(74) Representative: Krekora, Magdalena

(57) **Abstract**

A system for medical data storage and distribution contains at least one medical data recording device (4) containing data recorder module (1) and module of biometric sensor (2), which co-operate with each other in such a way, that biometric data of a person is linked with the collected data referring to this person, and it contains a module used for communication with an external data storage device, and additionally the system contains at least one data storage device (5) connected through a network or other similar means of communication (6) with the recording device (4), and where the recording device (4) sends data linked with identifying biometric data to the data storage device (5), where the data storage device (5) is equipped with software enabling access to the data after identification with biometric data.

## Description

### TECHNICAL FIELD

The object of the present invention is a medical data recording device and a system for medical data storage and distribution.

### BACKGROUND ART

Development of information technics made possible creation of medical databases. In case of this type of data it is particularly important to provide unambiguous identification and effective protection of access to them.

International patent application WO 2014/143950 discloses a computer system for collecting medical data together with biometric data, which grants access to medical data after identification with biometric data.

Patent application US 2014/0257833 discloses a remote medical checkup system, where medical data is remotely collected, stored in a cloud, and send to a medical services provider.

Patent application US 2014/0242698 discloses a system for medical data storage, where the data is connected with genetic code of the patient.

Patent application WO 2014/194939 discloses a method for verifying the identity of a user of an online service on the basis of biometrical parameters of the biometrical parameters per each face.

### DISCLOSURE OF INVENTION

A medical data recording device according to the present invention consists in that it contains data recording module and module of biometric sensor, which co-operate with each other in such a way, that biometric data of the person is linked with the collected data referring to this person, and the device contains module used for communication with an external data storage device.

A medical data recording device is an electronic device, which processes and/or stores medical data. The term "medical data" shall be understood as any digitized data of medical character being including medical signals or pictures. In particular following signals are medical signals: blood pressure, eye pressure, breath movements, body movements, force exerted by limbs, flow of fluids and gases, electrocardiography, electroencephalography, electromyography, electroneurography, magnetocardiography, magnetoencephalography, hormones in blood, chemical substances in blood or breath, laboratory analysis of feces or/and urine, audiometry, speech recording, body temperature, body weight, electronystagmography, spirometry, cardiotocography, and electrooculography. And medical pictures are photos (RTG, and other digital photos), tomography (MRI, SPECT, PET), ultrasonography (USG).

A device contains memory to store the data.

Module of biometric sensor is based on at least one method of biometric identification: fingerprints, hand palm geometry, voice timbre, eye iris image, eye retina image, face oval, DNA code, blood vessels system, ear shape, facial thermography, teeth contours and position, scent.

A device contains cryptographic module equipped with a key for calculation of MAC code (*Message Authentication Code),* where MAC code is calculated on the basis of data and identifying biometric data, then MAC code is allocated to biometric data linked with data.

A system for medical data storage and distribution according to the present invention consists in that it contains at least one medical data recording device containing data recording module and module of biometric sensor, which co-operate with each other in such a way, that biometric data of a person is linked with the collected data referring to this person, and it contains module used for communication with an external data storage device, and additionally the system contains at least one data storage device connected through a network or another similar means of communication with recording device, and where the recording device sends data linked with identifying biometric data to the data storage device, where the data storage device is equipped with software enabling access to the data after identification with biometric data.

Module of biometric sensor is based on at least one method of biometric identification: fingerprints, hand palm geometry, voice timbre, eye iris image, eye retina image, face oval, DNA code, blood vessels system, ear shape, facial thermography, teeth contours and position, scent.

The recording device is able to send biometric data and download data from the data storage device.

The recording device contains cryptographic module equipped with a key for calculation of MAC code (Message Authentication Code), where MAC code is calculated on the basis of data and identifying biometric data, then the recording device allocates MAC code to biometric data linked with data, and the data storage device verifies MAC code, and the data storage device is equipped with software enabling access to the data after identification with biometric data.

The recording device is able to send biometric data and download data from the data storage device, and to calculate MAC code, and MAC code is calculated by the recording device on the basis of biometric data, and the data from the data storage device may be downloaded by the recording device after positive verification of MAC code and biometric data.

Medical data together with their biometric identification are stored in the medical device or send to a given computer or data server (or another data storage device). Recorded medical data are biometrically identifiable during their processing, storage or/and sending. This method of medical data and biometric data collection may be compared to digital signature or digital stamp under the document which guarantees full and unambiguous data identification.

MAC code allocated to the medical data and bio-identification data authenticates sent data and ensures their integrity. MAC code is calculated by the device from the medical and biometric data using private and secret key stored in non-volatile memory of the device. Medical data receiving device (together with its biometric identification) has the same key as the sending device, what enables verification of MAC code received together with medical and biometric data. If the verification of MAC code by the receiving device is successful, it means that the sent medical data together with biometric identification of the patient were not altered or manipulated.

Medical data together with its biometric identification may be saved on selected computer or/and data server or/and other digital data storage device. Access to the data is possible in following manner:
a) locally by direct access of persons who operate the computer or/and data server or/and other medical data storage device,
b) remotely, data is sent to a local computer by a device (peripheral to the computer) used for biometric identification of a patient,
c) remotely, by a medical device with built-in sensor for biometric identification, which receives medical data sent from data server.

In cases b) and c) transfer of data stored in a server or/and other data storage device is effected on condition of prior biometric identification of a patient. A patient, who earlier sent medical data together with its biometric identification to the server, may download the medical data to the device or local computer only after identification on the basis of collected biometric data. It ensures remote, easy and safe access to the medical data by the owner of the medical data. Data sent from the server to the medical device may be utilized e.g. it may be viewed (visualized) on the screen of the device or sent to another local device (e.g. computer in doctor's office). Medical data sent from server to a medical device (case c) may or may not have attached MAC code. In case when data is read from the server, the server or a computer becomes a transmitter, and the medical device, used by the patient, is a receiver.

### BRIEF DESCRIPTION OF THE DRAWING

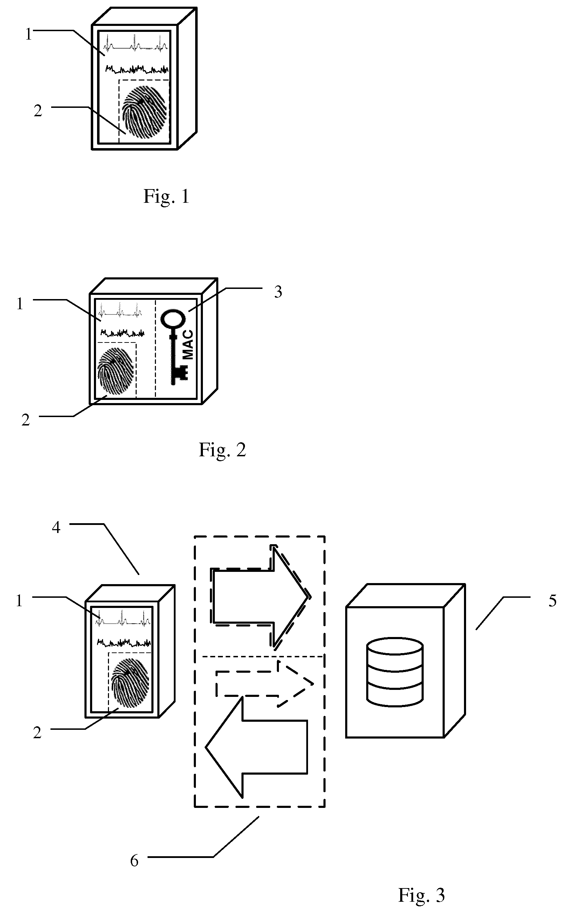

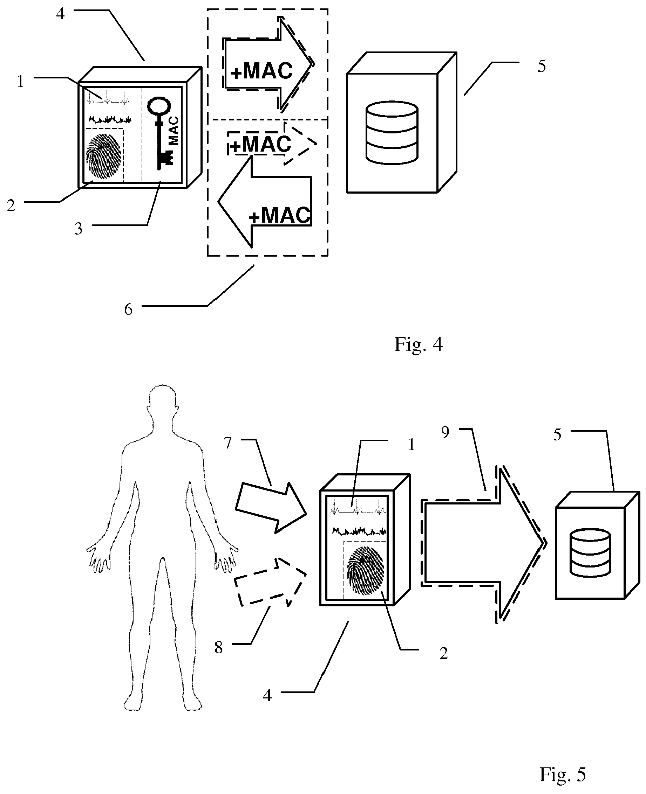

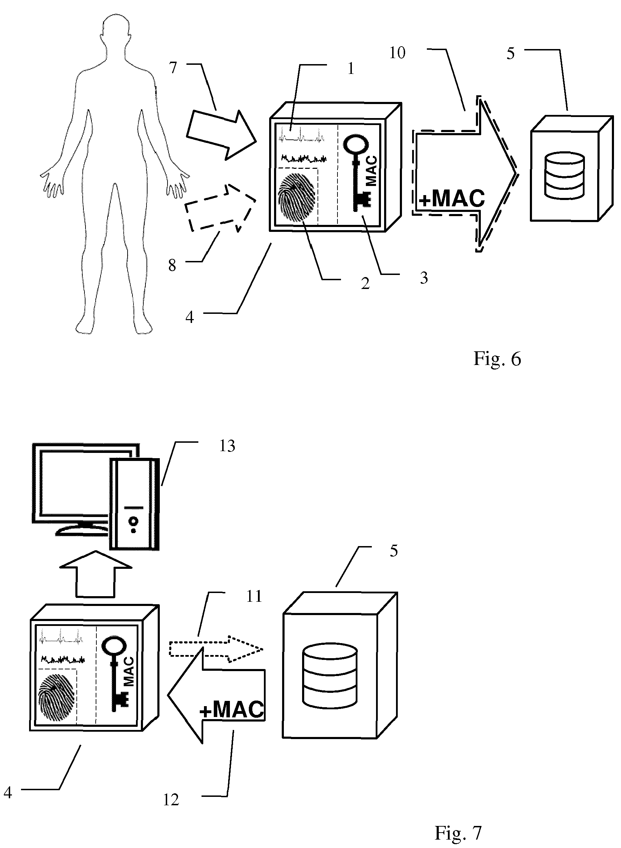

The device and the system according to the invention are disclosed at the drawings, where fig. 1 depicts a data recording device, fig. 2 depicts a data recording device with MAC code generator, fig. 3 depicts a system for medical data storage and distribution, fig. 4 depicts a system for medical data storage and distribution equipped additionally with cryptographic module, fig 5 depicts how the system operates, fig. 6 depicts how the system with cryptographic module operates, and fig. 7 depicts method of medical data receiving from the data storage device. All recording devices are equipped with a module for communication with an external data storage device.

### BEST MODE OF CARRYING OUT THE INVENTION

### Example 1

A medical data recording device contains data recording module 1 and module of biometric sensor 2. The data recording device is an ECG recording device equipped with fingerprint biometric sensor.

Before start of recording or during recording of ECG signal, and not later than before transfer of medical data, the patient places its finger on the fingerprint reader. As a result the recorded and transferred medical data is linked with Biometric Patient Identification (BPI). The ECG recording device is not equipped with non-volatile memory. In this case medical data together with BPI must be sent before the power source is discharged or changed. In this the results of the examinations must be sent to the computer or data server before next patient is examined.

### Example 2

A medical data recording device contains data recording module 1, module of biometric sensor 2, and cryptographic module 3. The data recording device is an ECG recording device equipped with fingerprint biometric sensor.

The ECG recording device has non-volatile memory (e.g. flashtype). In this case it is possible to exchange or recharge the power source without prior necessity to send medical data together with BPI to a computer of data server. It is possible because medical data together with BPI is stored in the non-volatile memory of the device. Additionally in this case the ECG recording device may be used for more than one patient, because before data recording or during data recording the patient is identified, and the medical data with BPI are saved in the memory. Transfer of medical data with BPI to a computer or data server may be perform between examinations of different patients or all results may be transferred after the end of all examinations. Owing to BPI for each result, medical data of all patients will be identifiable and easily separable. For integrity assurance and authentication of sent medical data with BPI the device attaches to the sent data - a MAC code, which is then verified by the receiving device (computer or data server). In order to calculate the MAC code both in the transmitting and receiving devices a secret, private cryptographic key is stored. In case of any alteration of the sent data, when the MAC verification is not successful, the computer or data server informs the operating personnel.

### Example 3

A medical device is equipped with electromyographic module used for diagnosis of electric activity of muscles and pulse oximeter for examination of blood oxygen saturation and patient's pulse. The device is equipped with biometric sensors: fingerprint reader, and face oval reader. In this case different medical signals and different methods of biometric identification are combined. Examined person may freely choose both the type of processed medical data, as well as the kind of biometric identification.
a. both electromyography and pulse oximetry,
b. only electromyography,
c. only pulse oximetry.

The same refers to the choice of method of biometric patient identification, where it is possible to choose:
a. identification of face oval and fingerprints,
b. identification of face oval only,
c. identification of fingerprints only.

Kind of tests and identification methods may be defined in the device memory.

### Example 4

Digital blood pressure measuring device is equipped with biometric sensor of eye iris reader. The device has non-volatile memory. Examined persons during a longer period of time e.g. several days carry out single tests of blood pressure. Before commencing each test each person identifies himself by biometric examination of eye iris. Medical data together with BPI is stored in the non-volatile memory. In this case there is no need (but of course there is such possibility) to send medical data to a computer or data server. Each pressure test is linked with BPI, what means that it is identifiable. Medical data together with BPI is stored in the non-volatile memory, so transfer of the results of tests with BPI to a computer or data server may be performed any time. In this case following scenarios are possible (depending on the setting of the device):
a. transfer of data for one person only (before the transfer the person is again identified by the device, in order to recognize whose data shall be sent),
b. transfer of all stored test results together with their biometric identification to a computer or data server.

### Example 5

A system for medical data storage and distribution contains a medical data recording device 4 containing data recording module 1, a module of biometric sensor 2, and a data storage device 5. The devices are connected through a network or another similar connection 6. The data recording device sends data linked with identifying biometric data to the data storage device. The data storage device is equipped with software enabling access to the data after identification with biometric data.

### Example 6

A system for medical data storage and distribution contains a medical data recording device 4 containing data recording module 1, a module of biometric sensor 2, a cryptographic module for MAC calculation 3 and a data storage device 5. The devices are connected through a network or another similar connection 6. The data recording device sends data linked with identifying biometric data to the data storage device. The data storage device is equipped with software enabling access to the data after identification with biometric data and after successful verification of MAC code.

### Example 7

A medical data recording device 4 equipped with a data recording module 1 and module of biometric sensor 2 records medical data 7 and biometric data 8, and sends medical data linked with biometric data 9 to the data storage device 5. The data storage device is equipped with software enabling access to the data after identification with biometric data.

### Example 8

A medical data recording device 4 equipped with a data recording module 1, a module of biometric sensor 2, and a cryptographic module 3 records medical data 7 and biometric data 8, and sends medical data linked with MAC code and with identifying biometric data 10 to the data storage device 5. The data storage device is equipped with software enabling access to the data after identification with biometric data, and after successful verification of MAC code.

### Example 9

A medical data recording device 4 - an ECG recording device with biometric patient identification on the basis of blood vessels system sends results of ECG test to a computer or data server. A physician during a control visit wants to download all medical data of the examined patient. The patient for the aim of identification uses a biometric sensor installed in the recording device. Biometric data 11, which unambiguously identifies the owner of the medical data, is sent from the recording device 4 for the data server. The data 12 (recorded ECG test results) together with MAC code are sent to the recording device. From the recording device they may be sent e.g. to a local computer 13. Medical data in order to be protected against alteration or manipulation is identified by MAC code. MAC code is calculated both by the data server transmitting the data, and by the medical device working as a receiver receiving data from the data server.

## Claims

1. A medical data recording device, **characterized in that** it contains data recording module (1) and module of biometric sensor (2), which co-operate with each other in such a way, that biometric data of the person is linked with the collected data referring to this person, and the device contains module used for communication with an external data storage device.

2. A device according to claim 1, **characterized in that** it contains memory to store the data.

3. A device according to claim 1 or 2, **characterized in that** module of biometric sensor (2) is based on at least one method of biometric identification: fingerprints, hand palm geometry, voice timbre, eye iris image, eye retina image, face oval, DNA code, blood vessels system, ear shape, facial thermography, teeth contours and position, scent.

4. A device according to claim 1 or 2 or 3, **characterized in that** it contains cryptographic module (3) equipped with a key for calculation of MAC code (*Message Authentication Code*), where MAC code is calculated on the basis of recorded data and identifying biometric data, then MAC code is allocated to biometric data linked with data.

5. A system for medical data storage and distribution, **characterized in that** it contains at least one medical data recording device (4) containing data recorder module (1) and module of biometric sensor (2), which co-operate with each other in such a way, that biometric data of a person is linked with the collected data referring to this person, and it contains a module used for communication with an external data storage device, and additionally the system contains at least one data storage device (5) connected through a network or other similar means of communication (6) with the recording device (4), and where the recording device (4) sends data linked with identifying biometric data to the data storage device (5), where the data storage device (5) is equipped with software enabling access to the data after identification with biometric data.

6. A system according to claim 5, **characterized in that** the module of biometric sensor (2) is based on at least one method of biometric identification: fingerprints, hand palm geometry, voice timbre, eye iris image, eye retina image, face oval, DNA code, blood vessels system, ear shape, facial thermography, teeth contours and position, scent.

7. A system according to claim 5 or 6, **characterized in that** the recording device (4) is able to send biometric data to and download data from the data storage device.

8. A system according to claim 5 or 6, **characterized in that** the recording device (4) contains a cryptographic module (3) equipped with a key for calculation of MAC code (*Message Authentication Code),* where MAC code is calculated on the basis of data and identifying biometric data, then the recording device (4) allocates MAC code to biometric data linked with data, and the data storage device (5) verifies MAC code, and the data storage device (5) is equipped with software enabling access to the data after identification with biometric data.

9. A system according to claim 7, **characterized in that** the recording device (4) is able to is able to send biometric data to and download data from the data storage device, and to calculate MAC code, and MAC code is calculated by the recording device on the basis of biometric data, and the data from the data storage device (5) may be downloaded by the recording device (4) after successful verification of MAC code and biometric data.
